(19) Europäisches Patentamt
European Patent Office
Office européen des brevets

(11) Publication number: **0 024 351**
**B1**

(12) # EUROPEAN PATENT SPECIFICATION

(45) Date of publication of patent specification: **31.10.84**

(51) Int. Cl.$^3$: **C 07 D 253/06,** A 61 K 31/53

(21) Application number: **80104841.4**

(22) Date of filing: **14.08.80**

(54) **Solid pharmaceutical formulation containing substitued phenyl-triazines.**

(30) Priority: **16.08.79 GB 7928641**

(43) Date of publication of application:
**04.03.81 Bulletin 81/09**

(45) Publication of the grant of the patent:
**31.10.84 Bulletin 84/44**

(84) Designated Contracting States:
**BE CH DE FR GB LI NL SE**

(56) References cited:
**GB-A- 759 014**

**THE JOURNAL OF THE AMERICAN SOCIETY, vol. 74, published June 20, 1952, USA G.H. HITCHINGS et al. "3,5-Diamino-AS-triazines as inhibitors of lactic acid bacteria and plasmodia". pages 3200-1**

**JOURNAL OF MEDICINAL CHEMISTRY, vol. 15, no. 8 published August 1972, R.W.A. REES e.a. "Antimalarial activities of some 3,5-diamino-as-triazine derivatives", pages 859-61**

(73) Proprietor: **THE WELLCOME FOUNDATION LIMITED**
**183-193 Euston Road**
**London NW1 2BP (GB)**

(72) Inventor: **Roth, Barbara**
**7 Lone Pine Road**
**Chapel Hill North Carolina 27514 (US)**
Inventor: **Miller, Alistaïr Ainslie**
**91 Elmshurst Gardens**
**Tonbridge Kent (GB)**
Inventor: **Sawyer, David Alan**
**60 Bourne Vale**
**Hayes Kent (GB)**

(74) Representative: **Berg, Wilhelm, Dr. et al**
**Patentanwälte Dr. Berg Dipl.-Ing. Stapf Dipl.-Ing. Schwabe Dr. Dr. Sandmair Postfach 86 02 45**
**Stuntzstrasse 16**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 024 351 B1**

**Description**

The present invention relates to a solid pharmaceutical formulation in unit dose form for use in humans containing from 20 to 2400 mg of substituted phenyl-triazines. The formulation is useful in medicine, especially in the treatment of CNS disorders, such as epilepsy.

UK Patent No. 759 014 discloses compounds of the formula (I):

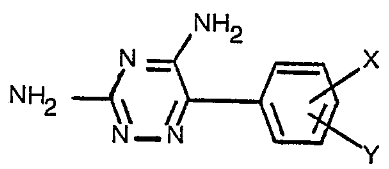

(I)

wherein X and Y are hydrogen and halogen atoms, as having activity against bacterial and malarial infections in animals. This patent specifically discloses those compounds wherein X and Y are both hydrogen atoms, wherein X is a hydrogen atom and Y is a 4-chloro atom and wherein X is a 4-chloro atom and Y is a 2-chloro and 3-chloro atom respectively. It shows no indication to the quantity of compounds which is necessary for the treatment of malarial infections in animals.

Rees *et al (J. Med. Chem.*, 1972, *15,* 859, have shown that these compounds, and in particular the 4-chlorophenyl, and the 3,4-dichlorophenyl compounds are active against the malaria organism *P. berghei* in mice. However, these compounds were also shown to be toxic and were not investigated further because of their low therapeutic ratio. The 2,4-dichlorophenyl compound had only slight anti-malarial activity. The therapeutic ratios of the compounds were such as to prevent their use in human medicine for the treatment or prophylaxis of malaria and they were not progressed further.

The minimum does of the 2,4-dichloro and the 2-chloro compounds were 40 mg/kg and 160 mg/kg respectively in mice. Even though it is appreciated that a strict correlation cannot be drawn between the dosage required in mice and man, these figures extrapolated would represent approximate doses of 2800 mg and 11200 mg in a 70 kg man. The actual dose administered would have to be at least twice this amount (see table II of the reference, minimum dose giving cure). In the Rees article referred to above, the 4-trifluoromethylphenyl compound was claimed to be less toxic than the chlorophenyl compounds whilst still being active against malaria. The other fluoro and trifluoromethyl compounds referred to in the article were substantially less active than the 4-trifluoromethylphenyl compound.

Rosenberg and Bottiroli *(Proc. Soc. exp, Biol.,* 1964 *115,* 410, described a series of tests in which three anti-malarial agents, quinacrine, chloroquine and hydroxychloroquine, were tested as anticonvulsants. Only hydroxychloroquine possessed a favourable activity profile.

In J. Amer. Chem. Soc. *74,* 3200—3201 (1952) G. H. Hitchings et al disclose 3,5-diamino-as-triazines as inhibitors of lactic acid bacteria and plasmodia. This document does not state anything about the minimum effective dose of such compounds in treatment of malaria.

It has now been found that a solid pharmaceutical formulation in unit dose form containing from 20 to 2400 mg of certain triazine compounds is useful in human medicine, especially for the treatment of CNS disorders, such as epilepsy.

Accordingly, the present invention provides a solid pharmaceutical formulation in unit dose form for use in humans containing from 20 to 2400 mg of 3,5-diamino-6-(2-chlorophenyl)-1,2,4-triazine, 3,5-diamino-6-(2,4-dichlorophenyl)-1,2,4-triazine or a pharmaceutically acceptable salt thereof optionally together with a pharmaceutically acceptable carrier.

Suitable acid addition salts of the active compounds include those formed with both organic and inorganic acids. Such acid addition salts will normally be pharmaceutically acceptable. Thus, preferred salts include those formed from hydrochloric, sulphuric, citric, tartaric, phosphoric, lactic, pyruvic, acetic, succinic, oxalic, fumaric, maleic, oxaloacetic acids, methanesulphoric, P-toluenesulphoric and benzenesulphoric acids.

The present invention provides a solid pharmaceutical formulation provided in discrete units, such as a tablet which contains an amount of the compounds, as hereinbefore defined, which is effective at such dosage or a multiple of the same, for instance such units contain 25 mg to 500 mg., usually around 50 mg. to 250 mg.

Other solid formulations in unit dose form include fine powders or granules which may contain diluting, dispersing and/or surface active agents and are formulated in capsules or sachets.

The solid formulations may contain and preferably will contain standard, pharmaceutically acceptable carriers for the purpose of administering the medicaments. Inert ingredients which may suitably be incorporated in the formulations of the present invention include lactose, starch, calcium phosphate, talc and magnesium stearate.

The formulations will be prepared by techniques well known to those skilled in the art. Thus, when a pharmaceutically acceptable carrier is present the solid formulations will be prepared by the admixture of an active compound with the pharmaceutically acceptable carrier. Conventional pharmaceutical excipients may be admixed as required.

As indicated above, the active triazines are generally useful in treating such disorders by oral administration.

The active compounds are normally administered at a dose of from 1 mg/kg to 30 mg/kg per day. The dose range for adult humans is generally from 20 mg to 2400 mg/day and preferably 350 to 1200 mg/day. Due to the fact that the active compounds are extremely long acting, it may often be advantageous to administer an initial dose of 70 to 2400 mg the first day and then a lower dose of 20 to 1200 mg on subsequent days.

The active triazines may be prepared by the method described in U.K. Patent No. 759 014 and U.S. Patent No. 3,637,688.

The anti-convulsant activity of the two compounds formulated in accordance with the present invention was determined by a standard maximal electroshock test, that is described by L. A. Woodbury and V. D. Devenport, *Arch. Int. Pharmacodyn.*, 1952, *92*, 97.

| | $ED_{50}$ mg/kg P.O. mice |
|---|---|
| 3,5-diamino-6-(2-chlorophenyl)-1,2,4-triazine | 10.5 |
| 3,5-diamino-6-(2,4-dichlorophenyl)-1,2,4-triazine | 18.7 |

The $LD_{50}$ (acute) of 3,5-diamino-6-(2-chlorophenyl)-1,2,4-triazine is approximately 1225 mg/kg p.o. in mice.

Tablet Example

| | | |
|---|---|---|
| 3,5-Diamino-6-(2,4-dichlorophenyl)-1,2,4-triazine | 150 mg | contents per tablet |
| Lactose | 200 mg | |
| Maize Starch | 50 mg | |
| Polyvinylpyrrolidone | 4 mg | |
| Magnesium Stearate | 4 mg | |

The drug was mixed with the lactose and starch and granulated with a solution of the polyvinylpyrrolidone in water. The resultant granules were dried, mixed with magnesium stearate and compressed to give tablets of average weight 408 mg.

**Claims**

1. A solid pharmaceutical formulation in unit dose form for use in humans containing from 20 to 2400 mg of 3,5-diamino-6-(2-chlorophenyl)-1,2,4-triazine, 3,5-diamino-6-(2,4-dichlorophenyl)-1,2,4-triazine or a pharmaceutically acceptable salt thereof.

2. A solid pharmaceutical formulation in unit dose form for use in humans containing from 20 to 2400 mg of 3,5-diamino-6-(2-chlorophenyl)-1,2,4-triazine, 3,5-diamino-6-(2,4-dichlorophenyl)-1,2,4-triazine or a pharmaceutically acceptable salt thereof according to claim 1 together with a pharmaceutically acceptable carrier.

**Patentansprüche**

1. Feste pharmazeutische Formulierung in Einheitsdosisform zur Verwendung bei Menschen, enthaltend 20 bis 2400 mg 3,5-Diamino-6-(2-chlorphenyl)-1,2,4-triazin, 3,5-Diamino-6-(2,4-dichlorphenyl)-1,2,4-triazin oder ein pharmazeutisch verträgliches Salz davon.

2. Feste pharmazeutische Formulierung in Einheitsdosisform zur Verwendung bei Menschen, enthaltend 20 bis 2400 mg 3,5-Diamino-6-(2-chlorphenyl)-1,2,4-triazin, 3,5-Diamino-6-(2,4-dichlorphenyl)-1,2,4-triazin oder ein pharmazeutisch verträgliches Salz davon gemäß Anspruch 1 zusammen mit einem pharmazeutisch unbedenklichen Träger.

**Revendications**

1. Formulation pharmaceutique solide sous forme dosée unitaire à administrer à l'être humain, contenant 20 à 2400 mg de 3,5-diamino-6-(2-chlorophényl)-1,2,4-triazine, de 3,5-diamino-6-(2,4-dichlorophényl)-1,2,4-triazine ou d'un de leurs sels pharmaceutiquement acceptables.

2. Formulation pharmaceutique solide sous forme dosée unitaire à administrer à l'être humain contenant 20 à 2400 mg de 3,5-diamino-6-(2-chlorophényl)1,2,4-triazine, de 3,5-diamino-6-(2,4-dichlorophényl)-1,2,4-triazine ou d'un de leurs sels pharmaceutiquement acceptables suivant la revendication 1, outre un excipient pharmaceutiquement acceptable.